Europäisches Patentamt

(19) European Patent Office  (11) Publication number: **0 184 615**

Office européen des brevets  **B1**

(12) **EUROPEAN PATENT SPECIFICATION**

(45) Date of publication of patent specification: **26.07.89**  (51) Int. Cl.⁴: **G 01 N 33/50**

(21) Application number: **85110627.8**

(22) Date of filing: **23.08.85**

(54) **Solid phase receptor assay for antibiotics of the vancomycin class.**

(30) Priority: **30.10.84 GB 8427436**

(43) Date of publication of application:
**18.06.86 Bulletin 86/25**

(45) Publication of the grant of the patent:
**26.07.89 Bulletin 89/30**

(84) Designated Contracting States:
**AT BE CH DE FR GB IT LI LU NL SE**

(56) References cited:
**EP-A-0 101 912**
**EP-A-0 119 613**
**EP-A-0 122 969**
**EP-A-0 132 117**

**BIOLOGICAL ABSTRACTS, vol. 77, no. 11,
November 1984, no. 81496, Philadelphia, US;
H. PARK et al.: "Detection of soluble
peptidoglycan in urine after penicillin
administration", & INFECT IMMUN. 43(1): 139-
142, 1984**

(73) Proprietor: **GRUPPO LEPETIT S.P.A.
23, Via G. Murat
I-20159 Milano (IT)**

(72) Inventor: **Corti, Angelo
2, Via Vittime di Piazza della Loggia
I-24100 Curnasco (Bergamo) (IT)**
Inventor: **Borghi, Angelo
36, Via Pierluigi da Palestrina
I-20124 Milano (IT)**
Inventor: **Rurali, Carlo
19, Viale Vittoria
I-20058 Villa Santa (Milano) (IT)**
Inventor: **Cassani, Giovanni
3, Via Vittadini
I-27100 Pavia (IT)**
Inventor: **Parenti, Francesco
8, Via Emilio De Marchi
I-20125 Milano (IT)**

Courier Press, Leamington Spa, England.

## Description

The present invention relates to a method for determining an antibiotic substance capable of binding to a D-alanyl-D-alanine dipeptide such as a glycopeptidic antibiotic of the Vancomycin class or a derivative or aglycone thereof. Further objects of the present invention are compositions of matter (kits) and means for exploiting the method of the invention.

Teicoplanin is the international non-proprietary name (INN) of the antibiotic substance formerly denominated teichomycin which was described in US Patent 4239751. Other antibiotics of the "vancomycin class" include, in addition to vancomycin, ristocetin, acetaplanin, teicoplanin, avoparcin, actinoidin, LL-AM-374, A 477, OA 7653, A 35512 B, A 515668, AAD 216, A 41030, A 47934 as well as the individual factors, derivatives and aglycones thereof.

It is known that vancomycin and ristocetin A interfere with the synthesis of peptidoglycan, which is the main structural component of the bacterial cell wall. It is thought that this interference, which leads to the inhibition of the cell growth and, eventually, to the destruction of the cell by lysis, is due to a specific binding between these antibiotics and the pentapeptide precursor having a D-Ala-D-Ala residue at the carboxyl terminus (UDP-N-Acetylmuramylpentapeptide) (H. R. Perkins, Biochem. J. 111, 195 (1969)).

It was recently discovered that also teicoplanin acts through the same mechanism by binding to the growing peptidoglycan of sensitive bacteria. More particularly, it was found that teicoplanin, like vancomycin and ristocetin A and similar antibiotics, binds to peptidoglycans containing a D-Ala-D-Ala dipeptide at the carboxy terminus. By so doing, they probably inhibit the bacterial transpeptidase and prevent the cross-linking of cell-wall peptidoglycan. These antibiotics also bind a D-alanyl-D-alanine dipeptide or a D-alanyl-D-alanine carboxy terminal oligopeptide.

Also the other antibiotics of the vancomycin class have proven or are supposed to act through the same mechanism of action, and are therefore capable of binding to a D-alanyl-D-alanine dipeptide or a D-alanyl-D-alanine carboxy terminal oligopeptide.

In addition to the antibiotics of the vancomycin class, the present method can suitably be used for determining other non-glycopeptidic or non-vancomycin-like antibiotics which are capable of binding to a D-alanyl-D-alanine dipeptide or a D-alanyl-D-alanine carboxy terminal oligopeptide.

D-alanyl-D-alanine or a D-alanyl-D-alanine oligopeptide can be considered therefore as a "molecular receptor" for the above described antibiotic substances.

Methods known so far for determining antibiotic substances and in particular teicoplanin and the other antibiotics of the vancomycin class are mainly based on TLC, HPLC, and bioassays on susceptible microorganisms (see for instance the cited US Patent).

In view of the current therapeutic use or advanced clinical study of some of these antibiotics, there is a need for assay methods for their determination in fluids, especially biological fluids, which be rapid, easy, reliable, and suitable for automation.

It has been found that it is possible to determine teicoplanin, and the other antibiotic substances (such as the other antibiotics of the vancomycin class) which have the same mechanism of action and bind to the same "molecular receptors" by means of a reliable, accurate, rapid and sensitive method which is based on competitive or saturation binding assays and employs a suitable conjugate form of a D-alanyl-D-alanine carboxy terminal oligopeptide adsorbed on a convenient solid phase.

The D-alanyl-D-alanine carboxy terminal oligopeptide may be a tri-, tetra-, penta-, hexa-, or heptapeptide wherein the carboxy terminal dipeptide is represented by D-alanyl-D-alanine. The preferred D-alanyl-D-alanine carboxy terminal oligopeptide of the invention is the tripeptide ε-aminocaproyl-D-alanyl-D-alanine (hereintofor: ε-Aca-D-Ala-D-Ala).

The D-alanyl-D-alanine carboxy terminal oligopeptide is conjugated with a suitable carrier in order to make it adsorbable on a solid phase surface. The suitable carrier may be in this case any protein or other high molecular weight substance capable of being covalently linked to the D-alanyl-D-alanine carboxy terminal oligopeptide to give a conjugate which is capable of adsorbing (i.e. capable of contracting *physical* bonds) with the selected solid phase surface.

A preferred example of such a carrier is albumine, and bovine albumine in particular. However egg, human, or pork albumine is usable as well.

The solid phase surface onto which the D-alanyl-D-alanine carboxy terminal oligopeptide-bearing carrier is adsorbed is preferably the surface of analytical supporting and/or containing means. For the scope of the present application "supporting and/or containing means" refers to any analytical means which can contain or, at least, be exposed to liquid reagents. Preferred examples of these are glass or plastic microtiter plates or test tubes or plastic sheets. The preferred "supporting and/or containing means" are the wells of polyethylene or polystyrene microtiter plates.

As already said, the "analyte", i.e. the substance that can be assayed by the method of the invention, is an antibiotic substance capable of binding to a D-alanyl-D-alanine dipeptide or a D-alanyl-D-alanine carboxy terminal oligopeptide, such as teicoplanin or another antibiotic substance of the vancomycin class (as defined above), an individual factor, derivative, aglycon or pseudo-aglycon thereof or another non-vancomycin like or non-glycopeptidic antibiotic substance which binds to the said "molecular receptor".

The method of the invention may be based on competitive or saturation binding assays.

As it is known, a competitive method is such that an analyte is quantitatively determined against a known amount of "labelled analyte" "in competition" for a limited number of "binding sites" for the analyte. A saturation method substantially differs from a competition one in that a two-step procedure is followed which comprises equilibrating the analyte with an excess of "binding sites" and then adding the "labelled analyte" in order to saturate all the binding sites which are still available.

As is apparent to the man skilled in the art, the "binding sites" in the method of the invention are represented by the so-called "molecular receptor" i.e. by the D-alanyl-D-alanine carboxy terminal oligopeptide conjugate adsorbed onto the surface of the analytical "supporting and/or containing means". "Labelled analyte" refer to an analyte which has been labelled by any of the known "labelling" means such as radioisotope tracers or preferably enzymes. The radioisotope tracers are those usually employed in radioimmunoassay such as $^{125}I$, while the enzymatic marker is one of those usually employed in enzyme-linked immunoassay. The preferred enzymatic-marker is horseradish peroxidase.

When the "labelled analyte" is an enzyme-labelled analyte the detection is preferably colorimetric in the presence of a suitable chromogenic substrate. In the case of radioisotopic labelling, the usual scintillographic detection techniques are used.

One object of the present invention is therefore a method for determining an analyte selected from an antibiotic of the vancomycin class, an individual factor, derivative or aglycon thereof; other glycopeptidic antibiotics, as well as non-glycopeptidic antibiotics, all of which possess the common feature of binding to the "molecular receptor" represented by a D-alanyl-D-alanine dipeptide or a D-alanyl-D-alanine carboxy terminal oligopeptide. This method includes passively adsorbing onto the plastic surface of the analytical "supporting and/or containing means", a determined amount of a D-alanyl-D-alanine carboxy terminal oligopeptide which has been conjugated with a macromolecular carrier capable of adsorbing onto said surface, adding either simultaneously or sequentially the test sample and a known amount of labelled analyte, and evaluating the amount of labelled analyte bound to the solid phase system by reference to a standard curve obtained by assaying known amounts of analyte.

It can be appreciated by those skilled in the art that the coupling of the D-alanyl-D-alanine carboxy terminal oligopeptide with the suitable carrier may be obtained by means of known per se techniques. Conveniently, a coupling agent is employed; a preferred coupling agent being glutaraldehyde. Other known coupling agents include: carbodiimides, diisocyanates, anhydrides, diazonium compounds, azides, and cyanogen bromide.

Similarly, known coupling procedures and reagents, such as those reported above, may be used also for linking the "labelling enzyme" to the analyte for preparing the "labelled analyte". Also in this case, glutaraldehyde is the preferred coupling agent.

In these coupling reactions, the proportion between the reactants may vary significantly, even if in many instances a ratio of about 1:1 between the "labelling enzyme" and the analyte, and between the D-alanyl-D-alanine carboxy terminal oligopeptide and carrier, respectively, is preferred. Moreover, in the case of the coupling reaction between a D-alanyl-D-alanine carboxy terminal oligopeptide which is ε-Aca-D-Ala-D-Ala and a macromolecular carrier which is bovine serum albumine (BSA) the molar proportion between these two reagents may vary from 1:1 to 10:1.

According to the method of the invention, a competitive assay includes:

a) passively adsorbing onto the surface of analytical "supporting and/or containing means" a D-alanyl-D-alanine carboxy terminal oligopeptide conjugated with a suitable macromolecular carrier capable of adsorbing onto said surfaces;

b) adding thereto, and incubating, the test sample together with a known amount of "labelled analyte";

c) evaluating the amount of analyte in the test sample by evaluating the amount of "labelled analyte" bound to the solid phase system by reference to a standard curve obtained with known amounts of analyte.

A preferred embodiment of the present invention is represented by the use of ε-Aca-D-Ala-D-Ala as the D-alanyl-D-alanine carboxy terminal oligopeptide in the method of the invention. Another preferred embodiment of the present invention is represented by the use of serum albumine, and preferably bovine serum albumine (BSA), as the macromolecular carrier capable of adsorbing onto plastic surfaces.

Still another preferred embodiment of the present invention is represented by the use of enzyme labelled analyte as the "labelled analyte"; the preferred "labelling" enzyme being horseradish peroxidase.

A further preferred embodiment of the method of the invention is represented by its use for determining an analyte which is selected from teicoplanin and a teicoplanin derivative, aglycon or pseudoaglycon.

A further specific embodiment of the invention is a competitive method for determining an analyte selected from teicoplanin, and another antibiotic substance of the vancomycin class, or an individual factor, derivative or aglycon thereof, in human serum samples, which comprises:

a) preparing a dilution buffer pH 7.0—7.5 with phosphate buffered saline (PBS), bovine serum albumine (BSA) and a surfactant agent such as a polyethylenoxide sorbitan mono-laurate (Tween® 20)

b) preparing scalar standard dilutions of the antibiotic to be tested (a zero standard included) in phosphate buffered saline (PBS) and diluted serum, pH 7.0—7.5,

c) diluting the test serum samples to the selected dilution by means of the above dilution buffer,

d) adding a solution of the horseradish peroxidase-analyte conjugate to each sample, mixing,

e) transferring each of these mixtures into the plastic wells of a microtiter plate coated with a limited amount of BSA-ε-Aca-D-Ala-D-Ala,

f) after having incubated for about 2 h at room temperature in a humid box, thoroughly washing the wells with PBS plus 0.05—0.1% (v/v) Tween® 20 pH 7.0—7.5,

g) adding a color developing chromogenic substrate for the horseradish peroxidase,

h) spectrophotometrically detecting the optical density of each sample, and

i) calculating the concentration of analyte in the test samples by interpolating the competition ratio of each sample on the calibration curve obtained with the standards.

The competition ratio is defined as the ratio between the specific optical density of the sample and the specific optical density of the zero standard.

The above procedure is referred to as the so-called "competition" binding assay, which is characterized by the fact that the total number of binding sites available is inferior to the sum of the analytes and labelled analytes for each sample.

However, this methodology can be modified and used in a "sequential saturation" assay which implies a two-step binding reaction.

In the first step the analyte contained in the standard or test sample is allowed to equilibrate with an excess of BSA-ε-Aca-D-Ala-D-Ala and, in the second step, after washing, the enzyme-labelled analyte is added to saturate the remaining binding sites. The bound enzymatic activity is revealed using the proper chromogenic substrate.

Analytical "supporting and/or containing" means coated with a D-alanyl-D-alanine carboxy terminal oligopeptide conjugated with a suitable macromolecular carrier capable of adsorbing onto said surface are new and represent a further object of the present invention. Also the plastic microtiter wells or tubes coated with a D-alanyl-D-alanine carboxy terminal oligopeptide conjugated with a suitable macromolecular carrier capable of adsorbing onto plastic surfaces are new and represent an object of the present invention.

Analogously, plastic microtiter wells or tubes coated with a BSA-ε-Aca-D-Ala-D-Ala conjugate are new and represent a further object and a preferred embodiment of the present invention.

More particularly, the preferred coated microtiter plates or tubes of the invention are plastic wells or tubes coated with from 0.025 μg to 1 μg preferably from 0.1 to 0.2 μg of BSA-ε-Aca-D-Ala-D-Ala.

The "supporting and/or containing means" of the invention are suitable for use in manual as well as automated assay procedures. This "flexibility" is of particular importance to meet the needs of the final user.

In fact, an automated procedure may be extremely useful in clinical monitoring of many treated patients both for keeping the therapeutic dose of the administered drug and for controlling or studying the drug distribution into the biological fluids, while a manual procedure may be sufficient for lab-scale analysis.

In its composition of matter aspect, the present invention encompasses also a kit of reagents for exploiting the method of the invention for the easy, rapid, reliable, and accurate determination of an analyte selected from an antibiotic of the vancomycin class, an individual factor, derivative or aglycon thereof, other glycopeptidic antibiotics, as well as non-glycopeptidic antibiotics all of which possess the common feature of binding to the "molecular receptor" represented by a D-alanyl-D-alanine dipeptide or a D-alanyl-D-alanine carboxy terminal oligopeptide, which includes:

a) analytical "supporting and/or containing means" coated with a D-alanyl-D-alanine carboxy terminal oligopeptide conjugated with a suitable macromolecular carrier capable of adsorbing onto plastic surfaces,

b) the labelled analyte and/or

c) a standard preparation of the analyte.

When the labelling substance is an enzyme, such as horseradish peroxidase, the kit may also contain a chromogenic substrate.

To the man skilled in the art, it is evident that no only other components may optionally be present in the "kit" such as buffer solutions and revealing means, but also some of the components of the kit may be presented in a separate form but for the same use. Also these separate forms of components for use in the present method are encompassed by the composition of matter and method of the present invention.

The following examples further illustrate the invention and are not to be construed as limiting its scope.

Example 1
Preparation of BSA-ε-ACA-D-ALA-D-ALA

a) Conjugation of ε-aminocaproyl-D-alanyl-D-Alanine (ε-Aca-D-Ala-D-Ala) to bovine serum albumine (BSA)

ε-Aca-D-Ala-D-Ala is conjugated to BSA by a "two step method" using glutaraldehyde as the bifunctional cross-linking reagent: BSA (250 mg) is dissolved in 0.1 M Na-carbonate/bicarbonate buffer at pH 9.5 (ml 4.5). Glutaraldehyde (0.5 ml of a 25% aqueous solution) is added and allowed to react for 1 hour at room temperature. The reaction mass is then dialyzed against 0.9% NaCl overnight. ε-Aca-D-Ala-D-Ala (125 mg) is dissolved in 0.5 ml of 0.5 M Na-carbonate buffer at pH 9.5, added to the dialyzed reaction mass and stirred for 6 h at room temperature. 1 M lysine (0.5 ml) is then added and the mixture is kept at room temperature for 2 h to block the unreacted active groups. The conjugate is then dialyzed against 0.05 M K-phosphate containing 0.15 M NaCl (PBS) pH 7.3.

b) Gel filtration of the conjugate

To purify the conjugate from excess unreacted material or low molecular weight by-products the above obtained dialyzed mixture is passed through a refrigerated Sephacryl® S-200 column (controlled pore covalently cross-linked alkyl dextrane and N,N'-methylene bis-acrylamide) (Pharmacia Fine Chemicals) 0.8 cm×100 cm) equilibrated and eluted with PBS pH 10.4 (flow rate=10 ml/h). The elution is monitored spectrophotometrically by registering the transmittance at 280 nm. Fractions corresponding to the peak eluted with the void volume of the column are pooled (total volume=15 ml), neutralized with 1 M HCl and diluted four times with PBS pH 7.3. Aliquots of 0.5 ml of this mixture are distributed into vials, lyophilized and kept at −80°C. The amount of conjugate prepared is sufficient for the coating of more than 10,000 microplates of 96 wells per microplate.

Example 2
Coating of microplates with BSA-ε-ACA-D-ALA-D-ALA

A stock solution for weekly preparation of microplates is prepared by dissolving the content of a vial of lyophilized BSA-ε-Aca-D-Ala-D-Ala (prepared as described in the foregoing example) in PBS pH 7.3 (0.5 ml); this solution can be stored for weeks without loss of activity and therefore suitable for use as stock solution for weekly preparations.

The wells of microplates are filled with 100 µl/well of a 1/2000 dilution of this stock solution, except one vertical row of wells (conveniently the last one) which is kept as the blank. These blank wells are filled with 100 µl of PBS pH 7.3. The microtiter plates are incubated for 3 hours at room temperature in a covered box and then washed 5 times by emptying and filling with distilled water. 200 µl/wells of 3% BSA in PBS pH 7.3 were added and allowed to adsorb for 2 hours at room temperature for blocking uncoated plastic surface. The plates are washed again with distilled water, shaken dry and stored at 4°C until use.

Example 3
Preparation of HRP-teicoplanin
a) Labelling of teicoplanin with horseradish peroxidase (HRP)

Teicoplanin is labelled with horseradish peroxidase by a "two step method" using glutaraldehyde as the bifunctional cross-linking reagent.

Teicoplanin (20 mg) in 0.1 M Na-carbonate pH 11.5 (2 ml) is added to 25% glutaraldehyde aqueous solution (0.2 ml). The mixture is neutralized with 1 M HCl and extensively dialyzed against 0.9% NaCl (at least 3 changes). Horseradish peroxidase (10 mg) in 0.5 M Na-carbonate/bicarbonate buffer pH 9.5 (0.5 ml) is added and stirred for 3 hours at 37°C. Then 1 M lysine (0.5 ml) is jdded thereto to block the unreacted active groups (15 hours at 4°C).

b) Gel filtration of HRP-teicoplanin

1.5 ml of the HRP-teicoplanin conjugate obtained as above is adjusted to pH 11 and gel-filtered on a refrigerated Sephacryl® S-200 column (0.8 cm×100 cm) pre-equilibrated in PBS pH 11 and eluted with the same buffer at a flow rate of 20 ml/h. The elution is monitored spectrophotometrically by registering the transmittance at 280 nm. Fractions of about 2.5 ml are collected at 4°C. Peak fractions (fractions which exhibit the maximum colorimetric development when assayed with the proper chromogenic reagent) are pooled and immediately neutralized with 1 M HCl.

c) Affinity chromatograph of HRP-teicoplanin

The above obtained fractions are further purified by affinity chromatography on a D-alanyl-D-alanine-ε-aminocaproyl-Sepharose® column (0.8 cm×10 cm) pre-equilibrated with PBS pH 7.3. After loading at a flow rate of 40 ml/hour, the column is washed with the equilibration buffer and eluted with PBS pH 11. The elution is monitored spectrophotometrically by registering the transmittance at 280 nm, fractions of 2 ml are collected. Peak fractions are pooled, neutralized with 1 M hydrochloric acid and tested for the enzymatic activity of the Teicoplanin-HRP conjugate. The most active fractions are aliquoted and stored at −80°C.

d) Evaluation of HRP-teicoplanin activity

Serial dilutions of the above obtained HRP-teicoplanin preparations are made in PBTBA buffer (PBS pH 7.3 containing 3% BSA, 0.5% Tween® 20, 10 mM benzamidine and 2 mg/ml 8-anilino-1-naphthalenesulfonic acid) 100 µl/well of each sample dilution is added to the horizontal rows of BSA-ε-Aca-D-Ala-D-Ala coated microplates and incubated for 2 hours in a covered box at room temperature. The microplates are washed eight times by emptying and filling with PBS pH 7.3 containing 0.05% Tween® 20 and shaken dry. 200 µl of a chromogenic solution (1 mg/ml o-phenylenediamine, 5 mM $H_2O_2$ in 0.1 M Na-citrate buffer at pH 5) is added to each well. After 30 min at room temperature, the enzymatic reaction is stopped by adding 50 µl/well of 4.5 M $H_2SO_4$. 15 min later, the optical density of each well at 492 nm is measured. The activity of the samples is expressed as the dilution factor giving 0.100—0.200 optical density units.

Example 4
Assay of teicoplanin containing serum samples
a) Preparation of dilution buffer

To 100 ml of phosphate buffered saline (PBS) pH 7.3, 200 mg of 8-anilino-1-naphthalenesulfonic acid (ANSA), 0.5 ml of Tween® 20 and 3 g of bovine serum albumin are added. This mixture is gently stirred until dissolution then the pH is adjusted to 7.3.

b) Preparation of diluted test serum

1 ml of human serum is diluted with 9 ml of the above dilution buffer.

c) Preparation of standard solution

Samples of about 5 mg of teicoplanin (accurately weighed) are put into glass test-tubes, 0.5 ml of dimethylformamide is added thereto and the mixture is dissolved by shaking or sonicating. Then, PBS at pH 7.3 is added to reach the concentration of 2 mg/ml. The solution is then diluted with PBS pH 7.3 to obtain the following dilutions of teicoplanin: 8, 4, 2, 1, 0.5, 0.25, 0.125, 0.062, 0.31 µg/ml. Diluted human serum is the zero standard.

d) Preparation of the solution of HRP-teicoplanin

HRP-teicoplanin, obtained as described in Example 3 is diluted 1 to 70 with the dilution buffer.

e) Preparation of the chromogenic solution 20 mg of o-phenylenediamine in 20 ml of 0.1 M Na-citrate buffer pH 5 is added to 10 µl of $H_2O_2$ 36% (w/v).

f) Preparation of the test samples

Serum or plasma samples are diluted 1:10 with the dilution buffer.

g) Assay procedure

Each standard solution (0.5 ml), zero solution included, as well as each test sample (0.5 ml) are transferred to plastic test tubes. A solution of HRP-teicoplanin as prepared above (0.5 ml) is added thereto and mixed. 100 µl of each of these solutions are then put in triplicate into the wells of a BSA-ε-Aca-D-Ala-D-Ala coated microtiter plate prepared as described in Example 2. Zero standard solutions are added to the "blank wells" of the microtiter plate. The plates are then incubated for 2 h at room temperature in a humid box, and washed thoroughly, 8 times, by emptying and filling with PBS containing 0.05% Tween® 20. Then the microplates are shaken dry. 200 µl/well of the above mentioned chromogenic solution is then added. After 30 min the color-developing enzymatic reaction is stopped by adding 50 µl/well of 4.5 M $H_2SO_4$. 15 min later the optical density of each well is measured at 492 nm.

h) Evaluation of the results

The mean values of the optical densities of the blank, the standard solutions and the test samples are separately evaluated. After subtracting the optical density value of the blank from the standard as well as the test samples, the competition ratio of each sample is determined by dividing each of the so obtained optical density values by the optical density of the zero standard. The teicoplanin content of the test samples is obtained by interpolating the corresponding competition ratio values on the calibration curve. The calibration curve is conveniently drawn on a logit-log paper plotting the competition ratio of each standard sample against the corresponding teicoplanin concentration.

Application range

(On the basis of repeated experiments): from 0.05 to 40 µg/ml.

Preferred application range from 0.1 to 30 µg/ml of teicoplanin.

Precision

—within the assay: CV% equal to 7.2.

—between the assays: CV% equal to 11.2.

Accuracy

—recovery ranged from 87 to 125%.

**Claims for the Contracting States: BE, CH, DE, FR, GB, IT, LI, LU, NL, SE**

1. A method for determining an analyte selected from an antibiotic of the vancomycin class, an individual factor, derivative or aglycon thereof; other glycopeptidic antibiotics, as well as non-glycopeptidic antibiotics, all of which possess the common feature of binding to the "molecular receptor" represented by a D-alanyl-D-alanine dipeptide or a D-alanyl-D-alanine carboxy terminal oligopeptide, which includes passively adsorbing onto the surface of analytical "supporting and/or containing means" a determined D-alanyl-D-alanine carboxy terminal oligopeptide conjugated with a macromolecular carrier capable of adsorbing onto said surface, adding either simultaneously or sequentially the test sample, and a known amount of labelled analyte, and evaluating the amount of labelled analyte bound to the solid phase system by reference to a standard curve obtained by assaying known amounts of analyte.

2. A method according to claim 1 wherein the D-alanyl-D-alanine carboxy terminal oligopeptide conjugate is in a limited amount compared with the total amount of analyte and labelled analyte and the test sample and the known amount of labelled analyte are added simultaneously to the adsorbed solid-phase.

3. A method according to claim 1 wherein the binding sites of the D-alanyl-D-alanine carboxy terminal oligopeptide conjugate exceed the amount of test analyte, the test analyte and the labelled analyte are added sequentially nd the amount of labelled analyte is such that it binds all the available binding sites of said adsorbed conjugate.

4. A method according to claim 1 wherein the analyte is an antibiotic substance of the vancomycin class selected from vancomycin, ristocetin, teicoplanin, actaplanin, avoparcin, actinoidin, LL-AM-374, A 477, OA 7653, A 35512 B, A 515668, AAD 216, A 41030, A 47934 as well as an individual factor, derivative, aglycon, thereof.

5. A method according to claim 1 wherein the analyte is teicoplanin.

6. A method according to claim 1 wherein the D-alanyl-D-alanine carboxy terminal peptide is ε-aminocaproyl-D-alanyl-D-alanine.

7. A method according to claim 1 wherein the macromolecular carrier is a protein.

8. A method according to claim 1 wherein the macromolecular carrier is albumin.

9. A method according to claim 1 wherein the analytical "supporting and/or containing means" are the wells of microtiter plates or test tubes, or plastic sheets.

10. A method according to claim 1 wherein the analytical "supporting and/or containing means" are the wells of polyethylene or polystyrene microtiter plates.

11. A method according to claim 1 wherein the labelling element of the "labelled analyte" is an enzyme.

12. A method according to claim 1 wherein the labelling element of the labelled analyte is horseradish peroxidase.

13. A method according to claim 1 which comprises:

a) preparing a dilution buffer pH 7.0—7.5 with phosphate buffered saline (PBS), bovine serum albumine (BSA) and a surfactant agent such as a polyethylene oxide sorbitan-mono-laurate (Tween® 20),

b) preparing scalar standard dilutions of the antibiotic to be tested (a zero standard included) in phosphate buffered saline (PBS) and diluted serum, pH 7.0—7.5,

c) diluting the test serum samples to the selected dilution by means of the above dilution buffer,

d) adding a solution of the horseradish peroxidase-analyte conjugate to each sample, mixing,

e) transferring each of these mixtures into the plastic wells of a microtiter plate coated with a limited amount of BSA-ε-Aca-D-Ala-D-Ala,

f) after having incubated for about 2 h at room temperature in a humid box, thoroughly washing the wells with PBS plus 0.05—0.1% (v/v) Tween® 20 pH 7.0—7.5,

g) adding a color developing chromogenic substrate for the horseradish peroxidase,

h) spectrophotometrically detecting the optical density of each sample, and

i) calculating the concentration of analyte in the test samples by interpolating the competition ratio of each sample on a calibration curve obtained with standard dilutions of the analytte.

14. Analytical "supporting and/or containing means" coated with a D-alanyl-D-alanine carboxy terminal oligopeptide conjugated with a suitable macromolecular carrier capable of adsorbing onto said surface for use in the method of claim 1.

15. Analytical means according to claim 14 wherein the D-alanyl-D-alanine carboxy terminal oligopeptide is ε-aminocaproyl-D-alanyl-D-alanine.

16. Analytical means according to claim 14 wherein the macromolecular carrier is a protein.

17. Analytical means according to claim 14 wherein the macromolecular carrier is albumin.

18. Analytical means according to claim 14 wherein said means are wells of microtiter plates or test tubes, or plastic sheets.

19. Analytical means according to claim 14 wherein said means are plastic microtiter plates, which are coated onto the internal surface of the wells with said conjugate.

20. A composition of matter (kit) for performing the method of claim 1 which includes:

a) analytical "supporting and/or containing means" coated with a D-alanyl-D-alanine carboxy terminal oligopeptide conjugated with a suitable macromolecular carrier capable of adsorbing onto said surface,

b) labelled analyte, and/or

c) standard preparation of the analyte.

21. A kit according to claim 20 wherein the D-alanyl-D-alanine carboxy terminal oligopeptide of the coated analytical "supporting and/or containing means" is ε-aminocaproyl-D-alanyl-D-alanine.

22. A kit according to claim 20 wherein the macromolecular carrier in the coated analytical "supporting and/or containing means" is a protein.

23. A kit according to claim 20 wherein the macromolecular carrier in the coated analytical "supporting and/or containing means" is albumin.

24. A kit according to claim 20 wherein said analytical "supporting and/or containing means" are wells of microtiter plates or test tubes.

25. A kit according to claim 20 wherein said analytical "supporting and/or containing means" are plastic wells of microtiter plates or test tubes.

## Claims for the Contracting State: AT

1. A method for determining an analyte selected from an antibiotic of the vancomycin class, an individual factor, derivative or aglycon thereof; other glycopeptidic antibiotics, as well as non-glycopeptidic antibiotics, all of which possess the common feature of binding to the "molecular receptor" represented by a D-alanyl-D-alanine dipeptide or a D-alanyl-D-alanine carboxy terminal oligopeptide, which includes passively adsorbing onto the surface of analytical "supporting and/or containing means" a determined D-alanyl-D-alanine carboxy terminal oligopeptide conjugated with a macromolecular carrier capable of adsorbing onto said surface, adding either simultaneously or sequentially the test sample, and a known amount of labelled analyte, and evaluating the amount of labelled analyte bound to the solid phase system by reference to a standard curve obtained by assaying known amounts of analyte.

2. A method according to claim 1 wherein the D-alanyl-D-alanine carboxy terminal oligopeptide conjugate is in a limited amount compared with the total amount of analyte and labelled analyte and the test sample and the known amount of labelled analyte are added simultaneously to the adsorbed solid-phase.

3. A method according to claim 1 wherein the binding sites of the D-alanyl-D-alanine carboxy terminal oligopeptide conjugate exceed the amount of test analyte, the test analyte and the labelled analyte are added sequentially and the amount of labelled analyte is such that it binds all the available binding sites of said adsorbed conjugate.

4. A method according to claim 1 wherein the analyte is an antibiotic substance of the vancomycin class selected from vancomycin, ristocetin, teicoplanin, actaplanin, avoparcin, actinoidin, LL-AM-374, A 477, OA 7653, A 35512 B, A 515668, AAD 216 A 41030, A 47934 as well as an individual factor, derivative, aglycon, thereof.

5. A method according to claim 1 wherein the analyte is teicoplanin.

6. A method according to claim 1 wherein the D-alanyl-D-alanine carboxy terminal peptide is epsilon-aminocaproyl-D-alanyl-D-alanine.

7. A method according to claim 1 wherein the macromolecular carrier is a protein.

8. A method according to claim 1 wherein the macromolecular carrier is albumin.

9. A method according to claim 1 wherein the analytical "supporting and/or containing means" are the wells of microtiter plates or test tubes, or plastic sheets.

10. A method according to claim 1 wherein the analytical "supporting and/or containing means" are the wells of polyethylene or polystyrene microtiter plates.

11. A method according to claim 1 wherein the labelling element of the "labelled analyte" is an enzyme.

12. A method according to claim 1 wherein the labelling element of the labelled analyte is horseradish peroxidase.

13. A method according to claim 1 which comprises:

a) preparing a dilution buffer pH 7.0—7.5 with phosphate buffered saline (PBS), bovine serum albumine (BSA) and a surfactant agent such as a polyethylene oxide sorbitan-mono-laurate (Tween® 20),

b) preparing scalar standard dilutions of the antibiotic to be tested (a zero standard included) in phosphate buffered saline (PBS) and diluted serum, pH 7.0—7.5,

c) diluting the test serum samples to the selected dilution by means of the above addition buffer,

d) mixing a solution of the horseradish, peroxidase-analyte conjugate to each sample, mixing,

e) transferring each of these mixtures into the plastic wells of a microtiter plate coated with a limited amount of BSA-epsilon-Aca-D-Ala-D-Ala,

f) after having incubated for about 2 h at room temperature in a humid box, thoroughly washing the wells with PBS plus 0.05—0.1% (v/v) Tween® 20 pH 7.0—7.5,

g) adding a color developing chromogenic substrate for the horseradish peroxidase,

h) spectrophotometrically detecting the optical density of each sample, and

i) calculating the concentration of analyte in the test samples by interpolating the competition ratio of each sample on a calibration curve obtained with standard dilutions of the analyte.

14. Analytical "supporting and/or containing means" coated with a D-alanyl-D-alanine carboxy terminal oligopeptide conjugated with a suitable macromolecular carrier capable of adsorbing onto said surface for use in the method of claim 1.

15. Analytical means according to claim 14 wherein the D-alanyl-D-alanine carboxy terminal oligopeptide is epsilon-aminocaproyl-D-alanyl-D-alanine.

16. Analytical means according to claim 14 wherein the macromolecular carrier is a protein.

17. Analytical means according to claim 14 wherein the macromolecular carrier is albumin.

18. Analytical means according to claim 14 wherein said means are wells of microtiter plates or test tubes, or plastic sheets.

19. Analytical means according to claim 14 wherein said means are plastic microtiter plates, which are coated onto the internal surface of the wells with said conjugate.

20. A process for the manufacture of a composition of matter (kit) for performing the method of claim 1 which includes:

a) analytical "supporting and/or containing means" coated with a D-alanyl-D-alanine carboxy terminal oligopeptide conjugated with a suitable macromolecular carrier capable of adsorbing onto said surface,

b) labelled analyte, and/or

c) standard preparation of the analyte.

21. A process according to claim 20 wherein the D-alanyl-D-alanine carboxy terminal oligopeptide of the coated analytical "supporting and/or containing means" is ε-aminocaproyl-D-alanyl-D-alanine.

22. A process according to claim 20 wherein the macromolecular carrier in the coated analytical "supporting and/or containing means" is a protein.

23. A process according to claim 20 wherein the macromolecular carrier in the coated analytical "supporting and/or containing means" is albumin.

24. A process according to claim 20 wherein said analytical "supporting and/or containing means" are wells of microtiter plates or test tubes.

25. A process according to claim 20 wherein said analytical "supporting and/or containing means" are plastic wells of microtiter plates or test tubes.

**Patentansprüche für die Vertragsstaaten: BE, CH, DE, FR, GB, IT, LI, LU, NL, SE**

1. Verfahren zur Bestimmung eines Analyts, das ein Antibiotikum der Vancomycin-Klasse, ein einzlener Faktor, ein Derivat oder Aglycon davon, ein anderes Glycopeptid-Antibiotikum oder ein nicht-glycopeptidisches Antibiotikum ist, wobei alle das gemeinsame Merkmal der Bindung an den durch ein D-Alanyl-D-Alanin-Dipeptid oder durch ein carboxyterminales D-Alanyl-D-Alanin-Oligopeptid dargestellten Rezeptor aufweisen, bei dem man an die Oberfläche eines analytischen "Trager- und/oder Aufnahme-Mittels" ein bestimmtes an einen markomolekularen Träger,

der an die Oberfläche absorbieren kann, konjungiertes carboxyterminales D-Alanyl-D-Alanin-Oligopeptid passiv absorbiert, gleichzeitig oder nacheinander die Probe und eine bekannte Menge markiertes Analyt zugibt, und die Menge des an das Festphasensystem gebundenen, markierten Analyts unter Bezugnahme auf eine durch Bestimmung bekannter Analytmengen erhaltene Eichkurve ermittelt.

2. Verfahren nach Anspruch 1, bei dem das carboxyterminale D-Alanyl-D-Alanin-Oligopeptid-Konjugat im Vergleich zur gesamten Mengen des Analyts und des markierten Analyts in beschränkter Menge vorliegt, und bei dem die Probe und die bekannte Menge des markierten Analyts gleichzeitig zur adsorbierten Festphase zugegeben werden.

3. Verfahren nach Anspruch 1, bei dem die Bindungsstellen am carboxyterminalen D-Alanyl-D-Alanin-Oligopeptid-Konjugat die Menge des zu bestimmenden Analyts übertreffen, das zu bestimmende Analyt und das markierte Analyt nacheinander zugegeben werden, und die Menge die markierten Analyts so groß ist, daß es alle verfügbaren Bindungsstellen des adsorbierten Konjugats bindet.

4. Verfahren nach Anspruch 1, bei dem das Analyt eines der Antibiotika der Vancomycin-Gruppe Vancomycin, Ristocetin, Teicoplanin, Actaplanin, Avoparcin, Actinoidin, LL-AM-374, A 477, OA 7653, A 35512 B, A 515668, AAD 216, A 41030, A 47934 oder ein einzlener Faktor, ein Derivat oder ein Aglycon davon ist.

5. Verfahren nach Anspruch 1, bei dem das Analyt Teicoplanin ist.

6. Verfahren nach Anspruch 1, bei dem das carboxyterminale D-Alanyl-D-Alanin-Peptid ε-Aminocaproyl-D-Alanyl-D-Alanin ist.

7. Verfahren nach Anspruch 1, bei dem der makromolekulare Träger ein Protein ist.

8. Verfahren nach Anspruch 1, bei dem der makromolekulare Träger Albumin ist.

9. Verfahren nach Anspruch 1, bei dem die analytischen "Trager- und/oder Aufnahme-Mittel" die Vertiefungen von Mikrotiterplatten oder Teströhrchen oder Kunststoffblätter sind.

10. Verfahren nach Anspruch 1, bei dem die analytischen "Träger- und/oder Aufnahme-Mittel" die Vertiefungen von Polyäthylen- oder Polystyrol-Mikrotiterplatten sind.

11. Verfahren nach Anspruch 1, bei dem die Markierung des "markierten Analyts" ein Enzym ist.

12. Verfahren nach Anspruch 1, bei dem die Markierung des markierten Analyts eine Meerrettich-Peroxidase ist.

13. Verfahren nach Anspruch 1, bei dem man folgende Schritte durchführt:

a) Herstellung eines Verdünnungspuffers mit einem pH-Wert von 7,0 bis 7,5 mit Phosphat-gepufferter Kochsalzlösung (PBS), Rinderserumalbumin (BSA) und einem Netzmittel, wie Polyäthylenoxidsorbitanmonolaurat (Tween® 20),

b) Herstellung skalarer Standardverdünnungen des zu bestimmenden Antibiotikums (einschließlich eines Nullwertes) in Phosphat-gepufferter Kochsalzlösung (PBS) und verdünntem Serum, pH 7,0 bis 7,5,

c) Verdünnung der zu bestimmenden Serumproben auf die gewünschte Verdünnung mit dem vorstehend genannten Verdünnungspuffer,

d) Zugabe einer Lösung des Meerrettich-Peroxidase-Analyt-Konjugats zu jeder Probe, Mischen,

e) Überführen jedes dieser Gemische in die Plastikvertiefungen einer Mikrotiterplatte, die mit einer begrenzten Menge BSA-ε-Aca-D-Ala-D-Ala beschichtet ist,

f) 2 Stunden Inkubation bei Raumtemperatur in einem feuchten Behältnis und anschließend sorgfältiges Waschen der Vertiefungen mit PBS und 0,05 bis 0,1% (Vol./Vol.) Tween® 20, pH 7,0 bis 7,5,

g) Zugabe eines ein Farbe bildenden chromogenen Substrates der Meerrettich-Peroxidase,

h) spektrophotometrischer Nachweis der optischen Dichte jeder Probe, und

i) Berechnung der Analytkonzentration in den zu bestimmenden Proben durch Interpolieren des Kompetitionsverhältnisses jeder Probe in einer Eichkurve, die mit Standardverdünnungen des Analyts erhalten wurde.

14. Analytisches "Trager- und/oder Aufnahme-Mittel", das mit einem an einen geeigneten makromolekularen Träger, der an die Oberfläche adsorbieren kann, konjugierten carboxyterminalen D-Alanyl-D-Alanin-Oligopeptid beschichtet ist, zur Verwendung im Verfahren nach Anspruch 1.

15. Analytisches Mittel nach Anspruch 14, in dem das carboxyterminale D-Alanyl-D-Alanin-Oligopeptid ε-Aminocaproyl-D-Alanyl-D-Alanin ist.

16. Aanlytisches Mittel nach Anspruch 14, in dem der makromolekulare Träger ein Protein ist.

17. Analytisches Mittel nach Anspruch 14, in dem der makromolekulare Träger Albumin ist.

18. Analytisches Mittel nach Anspruch 14, in dem das Mittel die Vertiefungen von Mikrotiterplatten oder Teströhrchen oder Kunststoffblätter sind.

19. Analytisches Mittel nach Anspruch 14, in dem das Mittel Kunststoff-Mikrotiterplatten sind, die auf der inneren Oberflächsr der Vertiefungen mit dem Konjugat beschichtet sind.

20. Zusammenstellung (Besteck) zur Durchführung des Verfahrens nach Anspruch 1, enthaltend:

a) analytische "Trager- und/oder Aufnahme-Mittel", die mit einem an einen geeigneten makromolekularen Träger, der an die Oberfläche adsorbieren kann, konjugierten carboxyterminalen D-Alanyl-D-Alanin-Oligopeptid beschichtet sind,

b) markiertes Analyt, und/oder

c) Standardpräparat des Aanlyts.

21. Besteck nach Anspruch 20, in dem das carboxyterminale D-Alanyl-D-Alanin-Oligopeptid des beschichteten analytischen "Träger- und/oder Aufnahme-Mittels" ε-Aminocapropyl-D-Alanyl-D-Alanin ist.

22. Besteck nach Anspruch 20, in dem der makromolekulare Träger im beschichteten analytischen "Träger- und/oder Aufnahme-Mittel" ein Protein ist.

23. Besteck nach Anspruch 20, in dem der makromolekulare Träger im beschichteten analytischen "Träger- und/oder Aufnahme-Mittel" Albumin ist.

24. Besteck nach Anspruch 20, in dem das analytische "Träger- und/oder Aufnahme-Mittel" die Vertiefungen von Mikrotiterplatten oder Teströhrchen sind.

25. Besteck nach Anspruch 20, in dem die analytischen "Träger- und/oder Aufnahme-Mittel" die Kunststoffvertiefungen von Mikrotiterplatten oder Teströhrchen sind.

**Patentansprüche für den Vertragsstaat: AT**

1. Verfahren zur Bestimmung eines Analyts, das ein Antibiotikum der Vancomycin-Klasse, ein einzelner Faktor, ein Derivat oder Aglycon davon, ein anderes Glycopeptid-Antibiotikum oder ein nicht-glycopeptidisches Antibiotikum ist, wobei alle das gemeinsame Merkmal der Bindung an den durch ein D-Alanyl-D-Alanin-Dipeptid oder durch ein carboxyterminales D-Alanyl-D-Alanin-Oligopeptid dargestellten Rezeptor aufweisen, bei dem man an die Oberfläche eines analytischen "Träger- und/oder Aufnahme-Mittels" ein bestimmtes an einen markomolekularen Träger, der an die Oberfläche adsorbieren kann, konjugiertes carboxyterminales D-Alanyl-D-Alanin-Oligopeptid passiv absorbiert, gleichzeitig oder nacheinander die Probe und eine bekannte Menge markiertes Analyt zugibt, und die Menge des an das Festphasensystem gebundenen, markierten Analyts unter Bezugsnahme auf eine durch Bestimmung bekannter Analytmengen erhaltene Eichkurve ermittelt.

2. Verfahren nach Anspruch 1, bei dem das carboxyterminale D-Alanyl-D-Alanin-Oligopeptid-Konjugat im Vergleich zur gesamten Menge des Analyts und des markierten Analyts in beschränkter Menge vorliegt, und bei dem die Probe und die bekannte Menge des markierten Analyts gleichzeitig zur adsorbierten Festphase zugegeben werden.

3. Verfahren nach Anspruch 1, bei dem die Bindungsstellen am carboxyterminalen D-Alanyl-D-Alanin-Oligopeptid-Konjugat die Menge des zu bestimmenden Analyts übertreffen, das zu bestimmende Analyt und das markierte Analyt nacheinander zugegeben werden, und die Menge des markierten Analyts so groß ist, daß es alle verfügbaren Bindungsstellen des adsorbierten Konjugats bindet.

4. Verfahren nach Anspruch 1, bei dem das Analyt eines der Antibiotika der Vancomycin-Gruppe Vancomycin, Ristocetin, Teicoplanin, Actaplanin, Avoparcin, Actinoidin, LL-AM-374, A 477, OA 7653, A 35512 B, A 515668, AAD 216, A 41030, A 47934 oder ein einzlender Faktor, ein Derivat oder ein Aglycon davon ist.

5. Verfahren nach Anspruch 1, bei dem das Analyt Teicoplanin ist.

6. Verfahren nach Anspruch 1, bei dem das carboxyterminale D-Alanyl-D-Alanin-Peptid ε-Aminocaproyl-D-Alanyl-D-Alanin ist.

7. Verfahren nach Anspruch 1, bei dem der makromolekulare Träger ein Protein ist.

8. Verfahren nach Anspruch 1, bei dem der makromolekulare Träger Albumin ist.

9. Verfahren nach Anspruch 1, bei dem die analytischen "Träger- und/oder Aufnahme-Mittel" die Vertiefungen von Mikrotiterplatten oder Teströhrchen oder Kunststoffblätter sind.

10. Verfahren nach Anspruch 1, bei dem die analytischen "Träger- und/oder Aufnahme-Mittel" die Vertiefungen von Polyäthylen- oder Polystyrol-Mikrotiterplatten sind.

11. Verfahren nach Anspruch 1, bei dem die Markierung des "markierten Analyts" ein Enzym ist.

12. Verfahren nach Anspruch 1, bei dem die Markierung des markierten Analyts eine Meerrettich-Peroxidase ist.

13. Verfahren nach Anspruch 1, bei dem man folgende Schritte durchführt:

a) Herstellung eines Verdünnungspuffers mit einem pH-Wert von 7,0 bis 7,5 mit Phosphatgepufferter Kochsalzlösung (PBS), Rinderserumalbumin (BSA) und einem Netzmittel, wie Polyäthylenoxidsorbitanmonolaurat (Tween® 20),

b) Herstellung skalarer Standardverdünnungen des zu bestimmenden Antibiotikums (einschließlich eines Nullwertes) in Phosphat-gepufferter Kochsalzlösung (PBS) und verdünntem Serum, pH 7,0 bis 7,5,

c) Verdünnung der zu bestimmenden Serumproben auf die gewünschte Verdünnung mit dem vorstehend genannten Verdünnungspuffer,

d) Zugabe einer Lösung des Meerrettich-Peroxidase-Analyt-Konjugats zu jeder Probe, Mischen,

e) Überführen jedes dieser Gemische in die Plastikvertiefungen einer Mikrotiterplatte, die mit einer begrenzten Menge BSA-ε-Aca-D-Ala-D-Ala beschichtet ist,

f) 2 Stunden Inkubation bei Raumtemperatur in einem feuchten Behältnis und anschließend sorgfältiges Waschen der Vertiefungen mit PBS und 0,05 bis 0,1% (Vol./Vol.) Tween® 20, pH 7,0 bis 7,5,

g) Zugabe eines ein Farbe bildenden chromogenen Substrates der Meerrettich-Peroxidase,

h) spektrophotometrischer Nachweise der optischen Dichte jeder Probe, und

i) Berechnung der Analytkonzentration in den zu bestimmenden Proben durch Interpolieren des Kompetitionsverhältnisses jeder Probe in einer Eichkurve, die mit Standardverdünnungen des Analyts erhalten wurde.

14. Analytisches "Träger- und/oder Aufnahme-Mittel", das mit einem an einen geeigneten makromolekularen Träger, der an die Oberfläche adsorbieren kann, konjugierten carboxyterminalen D-Alanyl-D-Alanin-Oligopeptid beschichtet ist, zur Verwendung im Verfahren nach Anspruch 1.

15. Analytisches Mittel nach Anspruch 14, in dem das carboxyterminale D-Alanyl-D-Alanin-Oligopeptid ε-Aminocaproyl-D-Alanyl-D-Alanin ist.

16. Analytisches Mittel nach Anspruch 14, in dem der makromolekulare Träger ein Protein ist.

17. Analytisches Mittel nach Anspruch 14, in dem der makromolekulare Träger Albumin ist.

18. Analytisches Mittel nach Anspruch 14, in dem das Mittel die Vertiefungen von Mikrotiterplatten oder Teströhrchen oder Kunststoffblätter sind.

19. Analytisches Mittel nach Anspruch 14, in dem das Mittel Kunststoff-Mikrotiterplatten sind, die auf der inneren Oberfläche der Vertiefungen mit dem Konjugat beschichtet sind.

20. Verfahren zur Herstellung einer Zusammenstellung (Besteck) zur Durchführung des Verfahrens nach Anspruch 1, enthaltend:

a) analytische "Träger- und/oder Aufnahme-Mittel", die mit einem an einen geeigneten makromolekularen Träger, der an die Oberfläche adsorbieren kann, konjugierten carboxyterminalen D-Alanyl-D-Alanin-Oligopeptid beschichtet sind,

b) markiertes Analyt, und/oder

c) Standardpräparat des Analyts.

21. Verfahren nach Anspruch 20, in dem das carboxyterminale D-Alanyl-D-Alanin-Oligopeptid des beschichteten analytischen "Träger- und/oder Aufnahme-Mittels" ε-Aminocapropyl-D-Alanyl-D-Alanin ist.

22. Verfahren nach Anspruch 20, in dem der makromolekulare Träger im beschichteten analytischen "Träger- und/oder Aufnahme-Mittel" ein Protein ist.

23. Verfahren nach Anspruch 20, in dem der makromolekulare Träger im beschichteten analytischen "Träger- und/oder Aufnahme-Mittel" Albumin ist.

24. Verfahren nach Anspruch 20, in dem das analytische "Träger- und/oder Aufnahme-Mittel" die Vertiefungen von Mikrotiterplatten oder Teströhrchen sind.

25. Verfahren nach Anspruch 20, in dem die analytischen "Träger- und/oder Aufnahme-Mittel" die Kunststoffvertiefungen von Mikrotiterplatten oder Teströhrchen sind.

**Revendications pour les etats contractants: BE, CH, DE, FR, GB, IT, LI, LU, NL, SE**

1. Procédé permettant de doser un analyte, choisi parmi les antibiotiques de la classe des vancomycines, leurs facteurs, dérivés ou aglycones individuels d'autres antibiotiques glycopeptidiques, ainsi que les antibiotiques non glycopeptidiques, qui présentent tous la caractéristique commune de se fixer au "récepteur moléculaire" représenté par un dipeptide D-alanyl-D-alanine ou par un oligopeptide D-alanyl-D-alanine carboxyterminal, qui consiste à faire adsorber d'une manière passive, par la surface d'un "moyen supportant et/ou contenant" analytique, un oligopeptide D-alanyl-D-alanine carboxy-terminal déterminé, conjugué à un support macromoléculaire capable d'être adsorbé par ladite surface, à ajouter, simultanément ou l'un après l'autre, l'échantillon d'essai et une quantité connue d'analyte marqué, et à évaluer la quantité de l'analyte marqué fixée au système en phase solide, par référence à une courbe étalon obtenue par l'analyse de quantités connues d'analyte.

2. Procédé selon la revendication 1, dans lequel le conjugué de l'oligopeptide D-alanyl-D-alanine carboxy-terminal est présent en une quantité limitée par rapport à la quantité totale de l'analyte et de l'analyte marqué, l'échantillon d'essai et la quantité connue de l'analyte marqué étant ajoutés simultanément à la phase solide adsorbée.

3. Procédé selon la revendication 1, dans lequel les sites de fixation du conjugué de l'oligopeptide D-alanyl-D-alanine carboxy-terminal dépassent la quantité de l'analyte d'essai, l'analyte d'essai et l'analyte marqué sont ajoutés l'un après l'autre et la quantité de l'analyte marqué est telle qu'il se fixe sur tous les sites de fixation disponibles dudit conjugué adsorbé.

4. Procédé selon la revendication 1, dans lequel l'analyte est une substance antibiotique de la classe des vancomycines, choisie parmi la vancomycine la ristocétine, la téicoplanine, l'actaplanine, l'avoparcine, l'actinoïdine, LL-AM-374, A 477, OA 7653, A 35512 B, A 515668, AAD 216, A 41030, A 47934, ainsi que leurs facteurs, dérivés ou aglycones individuels.

5. Procédé selon la revendication 1, dans lequel l'analyte est la téicoplanine.

6. Procédé selon la revendication 1, dans lequel le peptide D-alanyl-D-alanine carboxy-terminal est la ε-aminocaproyl-D-alanyl-D-alanine.

7. Procédé selon la revendication 1, dans lequel le support macromoléculaire est une protéine.

8. Procédé selon la revendication 1, dans lequel le support macromoléculaire est l'albumine.

9. Procédé selon la revendication 1, dans lequel les "moyens supportant et/ou contenants" analytiques sont les réservoirs de plaques de microtitrage ou de tubes à essai, ou des feuilles de matière plastique.

10. Procédé selon la revendication 1, dans lequel les "moyens supportants et/ou contenants" analytiques sont les réservoirs de plaques de microtitrage en polyéthylène ou polystyrène.

11. Procédé selon la revendication 1, dans lequel l'élément marqueur de "l'analyte marque" est une enzyme.

12. Procédé selon la revendication 1, dans lequel l'élément marqueur de l'analyte marqué est la peroxydase du raifort.

13. Procédé selon la revendication 1, qui consiste:

a) à préparer un tampon de dilution pH 7,0—7,5 avec une solution physiologique tamponnée par un tampon phosphate (PBS), l'albumine de sérum de boeuf (BSA) et un agent tensio-actif, comme le monolaurate de sorbitol polyoxyéthyléné (Tween® 20),

b) à préparer des dilutions étalon scalaires de l'antibiotique à essayer (y compris un étalon zéro) dans une solution physiologique tamponnée par un tampon phosphate (PBS) et du sérum dilué, pH 7,0—7,5,

c) à diluer les échantillons de sérum d'essai au rapport de dilution sélectionné, au moyen du tampon de dilution ci-dessus,

d) à ajouter à chaque échantillon, avant mélange, une solution du conjugué peroxydase du raifort-analyte,

e) à transférer chacun de ces mélanges dans les réservoirs de matière plastique d'une plaque de microtitrage revêtue d'une quantité limitée de BSA-ε-Aca-D-Ala-D-Ala,

f) après incubation pendant environ 2 heures à la température ambiante dans un caisson humide, à laver d'une manière approfondie les réservoirs à l'aide de PBS+0,05—0,1% (v/v) de Tween® 20, pH 7,0—7,5,

g) à ajouter un substrat chromogène développant la couleur, pour la peroxydase du raifort,

h) à détecter par spectrophotométrie la densité optique de chaque échantillon, et

i) à calculer la concentration de l'analyte dans les échantillons d'essai en interpolant le rapport de compétition de chaque échantillon sur une courbe d'étalonnage obtenue à l'aide des dilutions étalon de l'analyte.

14. "Moyen supportant et/ou contenant" analytique, revêtu d'un oligopeptide D-alanyl-D-alanine carboxy-terminal conjugué à un support macromoléculaire convenable, capable d'être adsorbé par ladite surface, pour utilisation dans le procédé de la revendication 1.

15. Moyen analytique selon la revendication 14, dans lequel l'oligopeptide D-analyl-D-alanine carboxy-terminal est la ε-aminocaproyl-D-analyl-D-alanine.

16. Moyen analytique selon la revendication 14, dans lequel le support macromoléculaire est une protéine.

17. Moyen analytique selon la revendication 14, dans lequel le support macromoléculaire est l'albumine.

18. Moyen analytique selon la revendication 14, dans lequel ledit moyen est constitué des réservoirs de plaques de mcirotitrage ou de tubes à essai, ou encore de feuilles de matière plastique.

19. Moyen analytique selon la revendication 14, dans lequel lesdits moyens sont des plaques de microtitrage de matière plastique, qui sont revêtues, sur la surface interne des réservoirs, dudit conjugué.

20. Composition de matière (coffret) pour mettre en oeuvre le procédé de la revendication 1, et comprenant:

a) un "moyen supportant et/ou contenant" analytique, revêtu d'un oligopeptide D-alanyl-D-alanine carboxy-terminal conjugué à un support macromoléculaire convenable, capable d'être adsorbé par ladite surface,

b) un analyte marqué, et/ou

c) une préparation étalon de l'analyte.

21. Coffret selon la revendication 20, dans lequel l'oligopeptide D-alanyl-D-alanine carboxy-terminal du "moyen supportant et/ou contenant" analytique ainsi revêtu, est la ε-aminocaproyl-D-ananyl-D-alanine.

22. Coffret selon la revendication 20, dans lequel le support macromoléculaire du "moyen supportant et/ou contenant" analytique ainsi revêtu est une protéine.

23. Coffret selon la revendication 20, dans lequel le support macromoléculaire du "moyen supportant et/ou contenant" analytique ainsi revêtu est l'albumine.

24. Coffret selon la revendication 20, dans lequel ledit "moyen supportant et/ou contenant" analytique est constitué de réservoirs de plaques de microtitrage, ou de tubes à essai.

25. Coffret selon la revendication 20, dans lequel ledit "moyen supportant et/ou contenant" analytique est constitué de réservoirs de matière plastique, de plaques de microtitrage, ou de tubes à essai.

**Revendications pour l'etat contractant: AT**

1. Procédé permettant de doser un analyte, choisi parmi les antibiotiques de la classe des vancomycines, leurs facteurs, dérivés ou aglycones individuels, d'autres antibiotiques glycopeptidiques, ainsi que les antibiotiques non glycopeptidiques, qui présentent tous la caractéristique commune de se fixer au "récepteur moléculaire" représenté par un dipeptide D-alanyl-D-alanine ou par un oligopeptide D-alanyl-D-alanine carboxy-terminal, qui consiste à faire adsorber d'une manière passive, par la surface d'un "moyen supportant et/ou contenant" analytique, un oligopeptide D-alanyl-D-alanine carboxy-terminal déterminé, conjugué à un support macromoléculaire capable d'être adsorbé par ladite surface, à ajouter, simultanément ou l'un après l'autre, l'échantillon d'essai et une quantité connue d'analyte marqué, et à évaluer la quantité de l'analyte marqué fixée au système en phase solide, par référence à une courbe étalon obtenue par l'analyse de quantités connues d'analyte.

2. Procédé selon la revendication 1, dans lequel le conjugué de l'oligopeptide D-alanyl-D-alanine carboxy-terminal est présent en une quantité limitée par rapport à la quantité totale de l'analyte et de l'analyte marqué, l'échantillon d'essai et la quantité connue de l'analyte marqué étant ajoutés simultanément à la phase solide adsorbée.

3. Procédé selon la revendication 1, dans lequel les sites de fixation du conjugé de l'oligopeptide D-alanyl-D-alanine carboxy-terminal dépassent la quantité de l'analyte d'essai, l'analyte d'essai et l'analyte marqué sont ajoutés l'un après l'autre et la quantité de l'analyte marqué est telle qu'il se fixe sur tous les sites de fixation disponibles dudit conjugué adsorbé.

4. Procédé selon la revendication 1, dans lequel l'analyte est une substance antibiotique de la classe des vancomycines, choisie parmi la vancomycine, la ristocétine, la téicoplanine, l'actaplanine, l'avoparcine, l'actionoïdine, LL-AM-374, A 477, OA 7653, A 35512 B, A 515668, AAD 216, A 41030, A 47934, ainsi que leurs facteurs, dérivés ou aglycones individuels.

5. Procédé selon la revendication 1, dans lequel l'analyte est la téicoplanine.

6. Procédé selon la revendication 1, dans lequel le peptide D-alanyl-D-alanine carboxy-terminal est la ε-aminocaproyl-D-alanyl-D-alanine.

7. Procédé selon la revendication 1, dans lequel le support macromoléculaire est une protéine.

8. Procédé selon la revendication 1, dans lequel le support macromoléculaire est l'albumine.

9. Procédé selon la revendication 1, dans lequel les "moyens supportant et/ou contenants" analytiques sont les réservoirs de plaques de microtitrage ou de tubes à essai, ou des feuilles de matière plastique.

10. Procédé selon la revendication 1, dans lequel les "moyens supportants et/ou contentants" analytiques sont les réservoirs de plaques de microtitrage en polyéthylène ou polystyrène.

11. Procédé selon la revendication 1, dans lequel l'élément marqueur de l'"analyte marqué" est une enzyme.

12. Procédé selon la revendication 1, dans lequel l'élément marqueur de l'analyte marqué est la peroxydase du raifort.

13. Procédé selon la revendication 1, qui consiste:

a) à préparer un tampon de dilution pH 7,0—7,5 avec une solution physiologique tamponnée par un tampon phosphate (PBS), l'albumine de sérum de boeuf (BSA) et un agent tensio-actif, comme le monolaurate de sorbitol polyoxyéthyléné (Tween® 20),

b) à préparer des dilutions étalon scalaires de l'antibiotique à essayer y compris un étalon zéro) dans une solution physioloqique tamponnée par un tampon phosphate (PBS) et du sérum dilué, pH 7,0—7,5,

c) à diluer les échantillons de sérum d'essai au rapport de dilution sélectionné, au moyen du tampon de dilution ci-dessus,

d) à ajouter à chaque échantillon, avant mélange, une solution du conjugué peroxydase du raifort-analyte,

e) à transférer chacun de ces mélanges dans les réservoirs de matière plastique d'une plaque de microtitrage revêtue d'une quantité limitée de BSA-ε-Aca-D-Ala-D-Ala,

f) après incubation pendant environ 2 heures à la température ambiante dans un caisson humide, à laver d'une manière appropfondie les réservoirs à l'aide de PBS+0,05—0,1% (v/v) de Tween® 20, pH 7,0—7,5,

g) à ajouter un substrat chromogène développant la couleur, pour la peroxydase du raifort,

h) à détecter par spectrophotométrie la densité optique de chaque échantillon, et

i) à calculer la concentration de l'analyte dans les échantillons d'essai en interpolant le rapport de compétition de chaque échantillon sur une courbe d'étalonnage obtenue à l'aide des dilutions étalon de l'analyte.

14. "Moyen supportant et/ou contenant" analytique, revêtu d'un oligopeptide D-alanyl-D-alanine carboxy-terminal conjugué à un support macromoléculaire convenable, capable d'être adsorbé par ladite surface, pour utilisation dans le procédé de la revendication 1.

15. Moyen analytique selon la revendication 14, dans lequel l'oligopeptide D-alanyl-D-alanine car-boxy-terminal est la ε-aminocaproyl-D-alanyl-D-alanine.

16. Moyen analytique selon la revendication 14, dans lequel le support macromoléculaire est une protéine.

17. Moyen analytique selon la revendication 14, dans lequel le support macromoléculaire est l'albumine.

18. Moyen analytique selon la revendication 14, dans lequel ledit moyen est constitué des réservoirs de plaques de microtitrage ou de tubes à essai, en encore de feuilles de matière plastique.

19. Moyen analytique selon la revendication 14, dans lequel lesdits moyens sont des plaques de microtitrage de matière plastique, qui sont revêtues, sur la surface interne des réservoirs, dudit conjugué.

20. Procédé de fabrication d'une composition de matière (coffret) pour mettre en oeuvre le procédé de la revendication 1, comprenant:

a) un "moyen supportant et/ou contentant" analytique, revêtu d'un oligopeptide D-alanyl-D-alanine carboxy-terminal conjugué à un support macromoléculaire convenable, capable d'être adsorbé par ladite surface,

b) un analyte marqué, et/ou

c) une préparation étalon de l'analyte.

21. Procédé selon la revendication 20, dans lequel l'oligopeptide D-alanyl-D-alanine carboxy-terminal du "moyen supportant et/ou contenant" analytique ainsi revêtu, est la ε-aminocaproyl-D-alanyl-D-alanine.

22. Procédé selon la revendication 20, dans lequel le support macromoléculaire du "moyen supportant et/ou contenant" analytique ainsi revêtu est une protéine.

23. Procédé selon la revendication 20, dans lequel le support macromoléculaire du "moyen supportant et/ou contenant" analytique ainsi revêtu est l'albumine.

24. Procédé selon la revendication 20, dans lequel ledit "moyen supportant et/ou contenant" analytique est constitué de réservoirs de plaques de microtitrage, ou de tubes à essai.

25. Procédé selon la revendication 20, dans lequel ledit "moyen supportant et/ou contenant" analytique est constitué de réservoirs de matière plastique, de plaques de microtitrage, ou de tubes à essai.